# EUROPÄISCHE PATENTANMELDUNG

(11) **EP 1 795 520 A1**
(43) Veröffentlichungstag der Anmeldung: **13.06.2007**
(21) Anmeldenummer: 05026849.9
(22) Anmeldetag: 08.12.2005
(51) Int. Cl.: C07C 51/275, C07C 63/333, C07C 63/38

(54) **Verfahren zur Herstellung einer aromatischen Carbonsäure**

(71) Anmelder: Rütgers Chemicals GmbH, 47138 Duisburg (DE)
(72) Erfinder: Müller-Hauck, Friedrich, 25557 Bendorf (DE); Müller, Peter, 49808 Lingen (DE); Thomsen, Sven Arne, 45276 Essen (DE); Böttcher, Frank, 45133 Essen (DE)
(74) Vertreter: Schaad, Balass, Menzl & Partner AG

(57) **Zusammenfassung**

Die Erfindung betrifft ein Verfahren zur Herstellung einer aromatischen Carbonsäure der allgemeinen Formel I 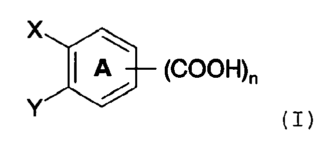 in der
X entweder eine Phenylgruppe ist und Y Wasserstoff bedeutet oder X und Y zusammen mit dem Benzolring A einen Naphthalinring bilden und
n eine ganze Zahl von 1 bis 4 ist, wobei sich die Carboxylgruppe(n) in einer beliebigen Stellung der beiden Benzolringe oder des Naphthalinrings befindet/befinden,
durch Oxidation eines entsprechenden Alkylaromaten.

Das Verfahren ist dadurch gekennzeichnet, dass ein isopropylsubstituierter Alkylaromat mit Salpetersäure oxidiert wird.

## Beschreibung

Die vorliegende Erfindung betrifft ein Verfahren zur Herstellung einer aromatischen Carbonsäure gemäss Anspruch 1 sowie die Verwendung von Salpetersäure zur Oxidation eines Alkylaromaten zur entsprechenden aromatischen Carbonsäure gemäss Anspruch 19.

Aromatische Carbonsäuren finden in vielerlei Gebieten der Industrie Anwendung und werden unter anderem zur Herstellung von Polyestern eingesetzt. In jüngerer Zeit haben insbesondere die aromatischen Carbonsäuren der allgemeinen Formel I an Bedeutung gewonnen: 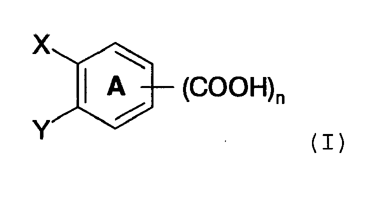 in der
X entweder eine Phenylgruppe ist und Y Wasserstoff bedeutet oder X und Y zusammen mit dem Benzolring A einen Naphthalinring bilden und
n eine ganze Zahl von 1 bis 4 ist, wobei sich die Carboxylgruppe(n) in einer beliebigen Stellung der beiden Benzolringe oder des Naphthalinrings befindet/befinden.

Wirtschaftlich besonders bedeutungsvolle Carbonsäuren der Formel I sind die 2,6-Naphthalindicarbonsäure und die 4,4'-Biphenyldicarbonsäure. So wird etwa die 2,6-Naphthalindicarbonsäure zur Herstellung des Polyesters Polyethylennaphthalat (PEN) verwendet. PEN ist Polyethylenterephthalat (PET) ähnlich, weist aber im Vergleich zu diesem wesentlich bessere Eigenschaften bezüglich Gasdurchlässigkeit und Temperaturbeständigkeit auf. PEN wird daher für Hochleistungskunststoffe eingesetzt. Die Herstellung von PEN ist aber teuer, was in erster Linie an der beschränkten Zugänglichkeit der Ausgangsverbindung 2,6-Naphthalindicarbonsäure liegt.

Konventionell werden aromatische Carbonsäuren, wie etwa die Terephthalsäure und die 2,6-Naphthalindicarbonsäure, durch Luftoxidation des entsprechenden Alkylaromaten in Essigsäure unter der Katalyse von Co/Mn und Bromid hergestellt (W. Partenheimer, Catalysis Today 23, 1995, 69-158). Während bei der Luftoxidation isopropylsubstituierter Aromaten zur entsprechenden aromatischen Carbonsäure die Ausbeuten selbst in der Gegenwart sehr hoher Katalysatormengen ungenügend sind, sind methylsubstituierte Aromaten, wie p-Xylol oder 2,6-Dimethylnaphthalin, für diese Verfahren gut geeignet und liefern relativ hohe Ausbeuten. Entsprechend kommen in den konventionellen Luftoxidationsverfahren aus wirtschaftlichen Überlegungen nur die methylsubstituierten Aromaten für die Herstellung der entsprechenden aromatischen Carbonsäure in Frage.

Der hauptsächliche Nachteil am konventionellen Luftoxidationsverfahren zur Herstellung aromatischer Carbonsäuren der allgemeinen Formel I liegt darin, dass die entsprechenden Ausgangsprodukte, d.h. die jeweiligen methylsubstituierten Aromaten, nur durch äusserst aufwendige, mehrstufige Verfahren darstellbar sind. So ist beispielsweise das 2,6-Dimethylnaphthalin über den Weg der Synthese nur sehr schwer zugänglich und entsprechend teuer.

Als Alternative zur Synthese wurde in EP-A-1264816 die Reinisolierung des 2,6-Dimethylnaphthalins aus den Dimethylnaphthalinisomerengemischen von Crackerfraktionen vorgeschlagen. Dieses Verfahren ist aber ebenfalls sehr aufwendig.

Zusätzlich zur schweren Zugänglichkeit des Ausgangsproduktes kommt beim konventionellen Luftoxidationsverfahren erschwerend hinzu, dass die Aufreinigung der dabei resultierenden aromatischen Carbonsäure ziemlich aufwendig ist. So lassen sich beispielsweise die bei der Luftoxidation von 2,6-Dimethylnaphthalin als Nebenprodukte entstehenden Naphthalinaldehyde und die halogenierten Naphthalincarbonsäuren nur sehr schwer von der gewünschten 2,6-Naphthalindicarbonsäure abtrennen.

Ein weiterer Nachteil des Luftoxidationsverfahrens ist die hohe notwendige spezifische Katalysatormenge und die Korrosivität der Bromidionen, welche aufwendige Sonderwerkstoffe erfordern.

Aufgabe der vorliegenden Erfindung ist es, ein Verfahren zur Herstellung einer aromatischen Carbonsäure der Formel I zur Verfügung zu stellen, das von einer leicht zugänglichen Ausgangsverbindung ausgeht, in der Abwesenheit von Katalysator unter schonenden Bedingungen abläuft und hohe Ausbeuten erzielt.

Die Aufgabe wird erfindungsgemäss gelöst durch ein Verfahren nach Anspruch 1. Bevorzugte Ausführungsformen der Erfindung sind in den unabhängigen Ansprüchen definiert.

Gemäss Anspruch 1 betrifft die Erfindung ein Verfahren zur Herstellung einer aromatischen Carbonsäure der allgemeinen Formel I 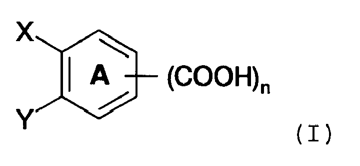 in der
X entweder eine Phenylgruppe ist und Y Wasserstoff bedeutet oder X und Y zusammen mit dem Benzolring A einen Naphthalinring bilden und
n eine ganze Zahl von 1 bis 4 ist, wobei sich die jeweilige Carboxylgruppe(n) in einer beliebigen Stellung der beiden Benzolringe oder des Naphthalinrings befindet/befinden,
durch Oxidation eines entsprechenden Alkylaromaten, dadurch gekennzeichnet, dass ein isopropylsubstituierter Alkylaromat mit Salpetersäure oxidiert wird.

Die aromatische Carbonsäure der Formel I weist zwei Ringe auf. Diese können kondensiert vorliegen und zusammen einen Naphthalinring bilden. Von der Formel I sind aromatische Carbonsäuren umfasst, in denen nur einer der beiden Ringe eine oder mehrere Carboxylgruppe(n) trägt, sowie aromatische Carbonsäuren, in denen beide Ringe eine oder mehrere Carboxylgruppe(n) tragen.

Der der aromatischen Carbonsäure der Formel I entsprechende Alkylaromat gemäss Anspruch 1 trägt die Alkylgruppe(n) in jener/n Stellung(en) der Ringe, in der/denen die Carbonsäure die Carboxylgruppe(n) trägt.

Gemäss der vorliegenden Erfindung ist der Alkylaromat ein isopropylsubstituierter Alkylaromat. Den obigen Ausführungen entsprechend trägt der isopropylsubstituierte Alkylaromat die Isopropylgruppe(n) in jener/n Stellung(en) der Ringe, in der/denen die herzustellende Carbonsäure die Carboxylgruppe(n) trägt. So ist beispielsweise der der 2,6-Naphthalindicarbonsäure entsprechende isopropylsubstituierte Alkylaromat das 2,6-Diisoproplynaphthalin.

Das erfindungsgemässe Verfahren hat den Vorteil, dass es von einer äusserst leicht zugänglichen Ausgangsverbindung ausgeht. So können beispielsweise isopropylsubstituierte Naphthaline aus den Alkylaten mit Propylen leicht durch Kristallisation in hoher Reinheit isoliert werden. In einer dem Fachmann bekannten Weise können die Verfahrensbedingungen bei der katalytischen Alkylierung des Naphthalins mit Propen derart gewählt werden, dass im resultierenden Isomerengemisch eines der Isomere in hoher Konzentration vorliegt. Im Gegensatz zu den Reinisomeren der methylierten Naphthaline sind die Reinisomere der isopropylierten Naphthaline, insbesondere das 2,6-Diisopropylnaphthalin, somit leicht zugänglich.

Das erfindungsgemässe Verfahren kann sowohl als Batchreaktion als auch in kontinuierlicher Fahrweise durchgeführt werden. Die Reaktionsgase werden bevorzugt unter Konstanthaltung des Druckes des Reaktionsraumes entspannt, wobei die nitrosen Gase zur Salpetersäure regeneriert werden können. Das Fehlen von Schwermetallkatalysatoren beim erfindungsgemässen Verfahren erleichtert das Reinigen des Reaktionsproduktes und die aufwendige Entfernung toxischer Schwermetalle aus dem Abwasser entfällt.

Da die Oxidation zudem in Abwesenheit von Halogenen abläuft, stellt sich beim erfindungsgemässen Verfahren auch das Problem der Abtrennung halogenierter aromatischer Carbonsäuren nicht.

Gegenüber der beispielsweise in DE-B-820308 und DE-B-2331082 beschriebenen Oxidation von Alkylgruppen an einkernigen aromatischen Systemen, die in Gegenwart hoher Salpetersäurekonzentrationen und bei hohen Temperaturen und Drucken durchgeführt werden, führt die Oxidation der Isopropylgruppen an den isopropylsubstituierten Alkylaromaten der vorliegenden Erfindung überraschenderweise bereits in stark verdünnter wässriger Salpetersäure und bei niedrigen Temperaturen und Drucken zu hohen Ausbeuten.

Die gemäss der vorliegenden Erfindung herzustellenden aromatischen Carbonsäuren tragen 1 bis 4 Carboxylgruppen. Bevorzugt wird eine aromatische Carbonsäure der Formel I hergestellt, in der beide Ringe mindestens eine Carboxylgruppe tragen.

In einer bevorzugten Ausführungsform des erfindungsgemässen Verfahrens wird eine aromatische Carbonsäure der Formel I hergestellt, in der X und Y zusammen mit dem Benzolring A einen Naphthalinring bilden und beide Ringe jeweils eine Carboxylgruppe tragen. Dabei wird besonders bevorzugt 2,6-Naphthalindicarbonsäure oder 1,5-Naphthalindicarbonsäure hergestellt.

In einer weiteren bevorzugten Ausführungsform des erfindungsgemässen Verfahrens wird eine aromatische Carbonsäure hergestellt, in der X eine Phenylgruppe ist, Y Wasserstoff bedeutet und beide Ringe jeweils eine Carboxylgruppe tragen. Dabei wird besonders bevorzugt 4,4'-Biphenyldicarbonsäure hergestellt. 2,6-Naphthalindicarbonsäure bzw. 4,4'-Biphenyldicarbonsäure sind technisch besonders bedeutungsvoll. Sie werden hauptsächlich zur Herstellung von Polyestern mit spezifischen Eigenschaften eingesetzt.

Ein weiteres bevorzugtes Verfahren ist dasjenige zur Herstellung von 4-Biphenylcarbonsäure.

Im allgemeinen beträgt die Salpetersäurekonzentration im erfindungsgemässen Verfahren 1,5 bis 7 Gewichts-%, bevorzugt 2,5 bis 5 Gewichts-% bezogen auf das Gesamtgewicht des Reaktionsgemisches.

Es ist bevorzugt, dass pro Isopropylgruppe des isopropylsubstituierten Alkylaromaten die 4 bis 10-fache stöchiometrische Menge an Salpetersäure, vorzugsweise die 5 bis 8-fache stöchiometrische Menge an Salpetersäure eingesetzt wird. Im allgemeinen wird wässrige Lösung von Salpetersäure eingesetzt. Vorzugsweise wird im Verfahren der vorliegenden Erfindung der isopropylsubstituierte Alkylaromat ausschliesslich mit Salpetersäure oxidiert.

Das Verfahren der vorliegenden Erfindung wird im allgemeinen bei einer Temperatur von 110°C bis 160°C, bevorzugt 130°C bis 150°C durchgeführt.

Niedrige Salpetersäurekonzentrationen und niedrige Temperaturen drängen insbesondere bei isopropylierten Alkylaromaten, die von Naphthalin abgeleitet sind, die Nebenreaktion der aromatischen Kernoxidation und die Nitrierung des Kerns zurück. So wird die Bildung des Nebenproduktes Trimellitsäure stark verringert, wenn das erfindungsgemässe Verfahren bei 130°C bis 150°C durchgeführt wird.

Ein weiterer Vorteil der milden Reaktionsbedingungen ist der niedrige Reaktionsdruck. Dieser liegt im allgemeinen im Bereich von 1 bis 40 bar, bevorzugt aber im Bereich von 2,5 bis 10 bar. Die Verwendung von teuren Hochdruckapparaten wird somit vermieden.

Das Verfahren gemäss der vorliegenden Erfindung wird anhand der Beispiele konkretisiert. Gemäss den Beispielen betrifft die vorliegende Erfindung insbesondere ein Verfahren zur Herstellung von 2,6-Naphthalindicarbonsäure, umfassend die nacheinanderfolgenden Schritte, dass
a) 2,6-Diisopropylnaphthalin mit wässriger Salpetersäure in einer Konzentration von ca. 5 Gewichts-% bezogen auf das Gesamtgewicht der Reaktionsmischung vorgelegt wird und
b) das Reaktionsgemisch gemäss a) auf ca. 140°C erhitzt wird,
um 2,6-Naphthalindicarbonsäure zu erhalten.

Bevorzugt wird dabei das 2,6-Diisopropylnaphthalin durch Alkylierung von Naphthalin mit Propen hergestellt. Die Reaktionsbedingungen können in einer dem Fachmann bekannten Weise gewählt werden, dass nach erfolgter Alkylierung das 2,6-Diisopropylnaphthalin-Isomere bis zu ca. 40 bis 50% bezogen auf das Gesamtgewicht der Reaktionsmischung vorliegt. Dieses kann in einem weiteren Schritt auf einfache Art und Weise kristallisiert und ausgefällt werden. Besonders hohe Ausbeuten an 2,6-Diisopropylnaphthalin werden erzielt, wenn die Propylierung bei einer Temperatur von 240 bis 260°C und die Transalkylierung bei einer Temperatur von 280 bis 300°C durchgeführt wird und der Druck auf 15 bis 20 bar eingestellt wird.

Weiter betrifft die Erfindung gemäss den Beispielen insbesondere ein Verfahren zur Herstellung von 4,4'-Biphenyldicarbonsäure, umfassend die nacheinanderfolgenden Schritte, dass
a) 4,4'-Diisopropylbiphenyl mit wässriger Salpetersäure in einer Konzentration von ca. 5 Gewichts-% bezogen auf das Gesamtgewicht der Reaktionsmischung vorgelegt wird und
b) das Reaktionsgemisch gemäss a) auf ca. 140°C erhitzt wird,
um 4,4'-Biphenyldicarbonsäure zu erhalten.

Wie in den Beispielen gezeigt wird, erfolgt die Oxidation des 4,4'-Diisopropylbiphenyls zur 4,4'-Biphenyldicarbonsäure in Gegenwart von Salpetersäure ebenfalls glatt und führt zu hohen Ausbeuten.

Entsprechend umfasst die vorliegende Erfindung auch die Verwendung von Salpetersäure zur Oxidation eines Alkylaromaten der Formel II 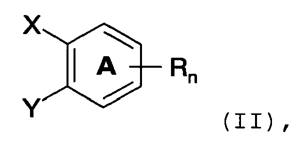 in der
X entweder eine Phenylgruppe ist und Y Wasserstoff bedeutet oder X und Y zusammen mit dem Benzolring A einen Naphthalinring bilden,
R eine Isopropylgruppe ist, die sich in einer beliebigen Stellung der beiden Benzolringe oder des Naphthaltinrings befindet, und
n eine ganze Zahl von 1 bis 4 ist
zur entsprechenden aromatischen Carbonsäure.

Dabei wird bevorzugt 2,6-Diisopropylnaphthalin oder 4,4'-Diisopropylbiphenyl oxidiert.

### Beispiele

### Beispiel 1

In einem Titanautoklaven werden 980 mg 2,6-Diisopropylnaphthalin und 78 ml 5-%ige wässrige Salpetersäure vorgelegt. Unter Magnetrührung wird der Inhalt auf 140°C erhitzt. Der Druck steigt auf ca. 28 bar. Nach vier Stunden wird die Reaktion abgebrochen. Die Salpetersäurekonzentration beträgt nach Reaktionsende 1,74%. Der ausgefallene Feststoff wird abfiltriert und durch ¹H-NMR Spektroskopie (300 MHz, Lösemittel d₆-DMSO) analysiert. Neben wenig nitrierten Naphthalinderivaten im Prozentbereich kann die 2,6-Naphthalindicarbonsäure als Hauptkomponente identifiziert werden: s 8,65 ppm (2H) d 8,21 bzw. 8,18 ppm (2H) und d 8,05 bzw. 8,02 ppm (2H). Das ¹³C-NMR-Spektrum zeigt die charakteristischen Signale (breitbandentkoppelt) bei:

| | |
|---|---|
| 167,17 -COOH | 2C |
| 134,13 | 2C |
| 130.13 | 4C |
| 129,68 | 2C |
| 125,88 | 2C |

Die Isolierausbeute an 2,6-Naphthalindicarbonsäure beträgt ca. 70%. Die Aufarbeitung der filtrierten wässrigen Phase ergibt Trimellitsäure:

### ¹H-NMR-Spektrum (d₆-DMSO, 300 MHz):

| | |
|---|---|
| s | 8,32 (1H) |
| d | 8,10 bzw. 8,08 |
| d | 7,87 bzw. 7,85 |

### Beispiel 2

Der gleiche Ansatz wie in Beispiel 1 wird bei 160°C Reaktionstemperatur durchgeführt. Dabei werden ca. 50% Isolierausbeute an 2,6-Naphthalindicarbonsäure erhalten. Es werden NMR-spektroskopisch wesentlich mehr nitrierte Naphthalinderivate im isolierten Feststoff gefunden als in Beispiel 1. In der wässrigen Phase wird auch wesentlich mehr Trimellitsäure gefunden als in Beispiel 1.

### Beispiel 3

In einen 300 ml Büchi-Glasautoklaven werden 60 ml 5%-ige wässrige Salpetersäure und 200 mg 4,4'-Diisopropylbiphenyl vorgelegt und auf ca. 140°C Innentemperatur während 6 h erhitzt. Gerührt wird mit einem Magnetrührer. Der Druck steigt auf 6 bar an. Nach dem Erkalten wird der ausgefallene Feststoff abfiltriert und NMR-spektroskopisch untersucht:

### ¹H-NMR (300 MHz, d₆-DMSO) :

| | | |
|---|---|---|
| d | 8,05 bzw. 8,02 | (4H) |
| d | 7,85 bzw. 7,82 | (4H) |

### ¹³C-NMR (breitbandentkoppelt):

| | |
|---|---|
| 166,99 (COOH) | 2C |
| 143,09 | 2C |
| 130,35 | 2C |
| 129,99 | 4C |
| 127,11 | 4C |

Nitrierte Nebenkomponenten sind in den Spektren nicht erkennbar. Die Isolierausbeute liegt bei ca. 90% an 4,4'-Biphenyldicarbonsäure.

## Patentansprüche

1. Verfahren zur Herstellung einer aromatischen Carbonsäure der allgemeinen Formel I 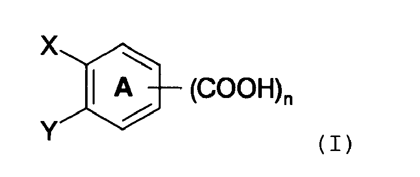 in der
X entweder eine Phenylgruppe ist und Y Wasserstoff bedeutet oder X und Y zusammen mit dem Benzolring A einen Naphthalinring bilden und
n eine ganze Zahl von 1 bis 4 ist, wobei sich die Carboxylgruppe(n) in einer beliebigen Stellung der beiden Benzolringe oder des Naphthalinrings befindet/befinden,
durch Oxidation eines entsprechenden Alkylaromaten, **dadurch gekennzeichnet, dass** ein isopropylsubstituierter Alkylaromat mit Salpetersäure oxidiert wird.

2. Verfahren nach Anspruch 1, **dadurch gekennzeichnet, dass** eine aromatische Carbonsäure der Formel I hergestellt wird, in der beide Ringe mindestens eine Carboxylgruppe tragen.

3. Verfahren nach einem der vorhergehenden Ansprüche, **dadurch gekennzeichnet, dass** eine aromatische Carbonsäure der Formel I herstellt wird, in der X und Y zusammen mit dem Benzolring A einen Naphthalinring bilden und beide Ringe jeweils eine Carboxylgruppe tragen.

4. Verfahren nach Anspruch 3, **dadurch gekennzeichnet, dass** 2,6-Naphthalindicarbonsäure oder 1,5-Naphthalindicarbonsäure hergestellt wird.

5. Verfahren nach Anspruch 1 oder 2, **dadurch gekennzeichnet, dass** eine aromatische Carbonsäure der Formel I hergestellt wird, in der X eine Phenylgruppe ist, Y Wasserstoff bedeutet und beide Ringe jeweils eine Carboxylgruppe tragen.

6. Verfahren nach Anspruch 5, **dadurch gekennzeichnet, dass** 4,4'-Biphenyldicarbonsäure hergestellt wird.

7. Verfahren nach Anspruch 1, **dadurch gekennzeichnet, dass** 4-Biphenylcarbonsäure hergestellt wird.

8. Verfahren nach einem der vorhergehenden Ansprüche, **dadurch gekennzeichnet, dass** die Konzentration von Salpetersäure 1,5 bis 7 Gewichts-% bezogen auf das Gesamtgewicht des Reaktionsgemisches beträgt.

9. Verfahren nach Anspruch 8, **dadurch gekennzeichnet, dass** die Konzentration von Salpetersäure 2,5 bis 5 Gewichts-% bezogen auf das Gesamtgewicht des Reaktionsgemisches beträgt.

10. Verfahren nach einem der vorhergehenden Ansprüche, **dadurch gekennzeichnet, dass** pro Isopropylgruppe des isopropylsubstituierten Alkylaromaten die 4 bis 10-fache stöchiometrische Menge an Salpetersäure eingesetzt wird.

11. Verfahren nach Anspruch 10, **dadurch gekennzeichnet, dass** pro Isopropylgruppe des isopropylsubstituierten Alkylaromaten die 5 bis 8-fache stöchiometrische Menge an Salpetersäure eingesetzt wird.

12. Verfahren nach einem der vorhergehenden Ansprüche, **dadurch gekennzeichnet, dass** es bei einer Temperatur von 110°C bis 160°C durchgeführt wird.

13. Verfahren nach Anspruch 12, **dadurch gekennzeichnet, dass** es bei einer Temperatur von 130°C bis 150°C durchgeführt wird.

14. Verfahren nach einem der vorhergehenden Ansprüche, **dadurch gekennzeichnet, dass** es bei einem Druck von 2.5 bis 10 bar durchgeführt wird.

15. Verfahren nach einem der vorhergehenden Ansprüche, **dadurch gekennzeichnet, dass** der isopropylsubstituierte Alkylaromat ausschliesslich mit Salpetersäure oxidiert wird.

16. Verfahren nach einem der vorhergehenden Ansprüche zur Herstellung von 2,6-Naphthalindicarbonsäure, umfassend die nacheinanderfolgenden Schritte, dass
a) 2,6-Diisopropylnaphthalin mit wässriger Salpetersäure in einer Konzentration von ca. 5 Gewichts-% bezogen auf das Gesamtgewicht der Reaktionsmischung vorgelegt wird und
b) das Reaktionsgemisch gemäss a) auf ca. 140°C erhitzt wird,
um 2,6-Naphthalindicarbonsäure zu erhalten.

17. Verfahren nach Anspruch 16, **dadurch gekennzeichnet, dass** das 2,6-Diisopropylnaphthalin durch Alkylierung von Naphthalin mit Propen hergestellt wird, wobei die Propylierung bei einer Temperatur von 240 bis 260°C und die Transalkylierung bei einer Temperatur von 280 bis 300°C durchgeführt wird und der Druck auf 15 bis 20 bar eingestellt wird.

18. Verfahren nach einem der vorhergehenden Ansprüche zur Herstellung von 4,4'-Biphenyldicarbonsäure, umfassend die nacheinanderfolgenden Schritte, dass
a) 4,4'-Diisopropylbiphenyl mit wässriger Salpetersäure in einer Konzentration von ca. 5 Gewichts-% bezogen auf das Gesamtgewicht der Reaktionsmischung vorgelegt wird und
b) das Reaktionsgemisch gemäss a) auf ca. 140°C erhitzt wird,
um 4,4'-Biphenyldicarbonsäure zu erhalten.

19. Verwendung von Salpetersäure zur Oxidation eines Alkylaromaten der Formel II 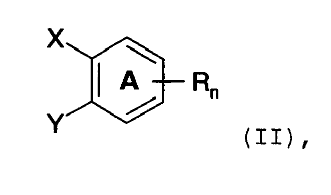 in der
X entweder eine Phenylgruppe ist und Y Wasserstoff bedeutet oder X und Y zusammen mit dem Benzolring A einen Naphthalinring bilden,
R eine Isopropylgruppe ist, die sich in einer beliebigen Stellung der beiden Benzolringe oder des Naphthalinringes befindet, und
n eine ganze Zahl von 1 bis 4 ist,
zur entsprechenden aromatischen Carbonsäure.

20. Verwendung nach Anspruch 19, **dadurch gekennzeichnet, dass** 2,6-Diisopropylnaphthalin oder 4,4'-Diisopropylbiphenyl oxidiert wird.
